(19) Europäisches Patentamt
European Patent Office
Office européen des brevets

(11) EP 1 639 119 B1

(12) **EUROPÄISCHE PATENTSCHRIFT**

(45) Veröffentlichungstag und Bekanntmachung des Hinweises auf die Patenterteilung:
**21.04.2010 Patentblatt 2010/16**

(21) Anmeldenummer: **04740018.9**

(22) Anmeldetag: **17.06.2004**

(51) Int Cl.:
*C12P 7/42* (2006.01)

(86) Internationale Anmeldenummer:
**PCT/EP2004/006564**

(87) Internationale Veröffentlichungsnummer:
**WO 2004/111257 (23.12.2004 Gazette 2004/52)**

(54) **VERFAHREN ZUR MIKROBIOLOGISCHEN ISOMERISIERUNG VON ALPHA-HYDROXYCARBONSÄUREN**

METHOD FOR THE MICROBIOLOGICAL ISOMERISATION OF ALPHA-HYDROXY CARBOXYLIC ACIDS

PROCEDE D'ISOMERISATION MICROBIOLOGIQUE D'ACIDES ALPHA-HYDROXYCARBOXYLIQUES

(84) Benannte Vertragsstaaten:
**AT BE BG CH CY CZ DE DK EE ES FI FR GB GR HU IE IT LI LU MC NL PL PT RO SE SI SK TR**

(30) Priorität: **18.06.2003 DE 10327582**

(43) Veröffentlichungstag der Anmeldung:
**29.03.2006 Patentblatt 2006/13**

(73) Patentinhaber: **BASF SE**
**67056 Ludwigshafen (DE)**

(72) Erfinder:
- **GLÜCK, Silvia**
  **A-8047 Graz (AT)**
- **SCHNELL, Barbara**
  **A-8010 Graz (AT)**
- **PIRKER, Monika**
  **A-8010 Graz (AT)**
- **FABER, Kurt**
  **A-8010 Graz (AT)**

(56) Entgegenhaltungen:
**EP-A- 0 596 466**

- **SILVIA M. GLÜCK ET AL.: "Lactate Racemase as a Versatile Tool for the Racemization of Alpha-Hydroxycarboxylic Acids" CHEMICKE LISTY, Bd. 97, Nr. 6, 1. Juni 2003 (2003-06-01), Seite 363, XP002301107 PRAHA, CZ**
- **SCHNELL, BARBARA ET AL: "Enzymatic racemization and its application to synthetic biotransformations" ADVANCED SYNTHESIS & CATALYSIS , 345(6+7), 653-666 CODEN: ASCAF7; ISSN: 1615-4150, 13. Juni 2003 (2003-06-13), XP002301108**
- **LIU S -Q: "Practical implications of lactate and pyruvate metabolism by lactic acid bacteria in food and beverage fermentations." INTERNATIONAL JOURNAL OF FOOD MICROBIOLOGY, Bd. 83, Nr. 2, 15. Juni 2003 (2003-06-15), Seiten 115-131, XP002301109 ISSN: 0168-1605**
- **STRAUSS U T ET AL: "Deracemization of (+-)-mandelic acid using a lipase-mandelate racemase two-enzyme system" TETRAHEDRON ASYMMETRY 1999 UNITED KINGDOM, Bd. 10, Nr. 21, 1999, Seiten 4079-4081, XP002301110 ISSN: 0957-4166**
- **FELFER ULFRIED ET AL: "Substrate spectrum of mandelate racemase. Part 2. (Hetero)-aryl-substituted mandelate derivatives and modulation of activity" JOURNAL OF MOLECULAR CATALYSIS B ENZYMATIC, Bd. 15, Nr. 4-6, 1. November 2001 (2001-11-01), Seiten 213-222, XP002301111 ISSN: 1381-1177 in der Anmeldung erwähnt**
- **SCHAFER SUSAN L ET AL: "Mechanism of the reaction catalyzed by mandelate racemase: Structure and mechanistic properties of the D270N mutant" BIOCHEMISTRY, Bd. 35, Nr. 18, 1996, Seiten 5662-5669, XP002301112 ISSN: 0006-2960 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- GARCIA-VILOCA M ET AL: "A QM/MM study of the racemization of vinylglycolate catalyzed by mandelate racemase enzyme." JOURNAL OF THE AMERICAN CHEMICAL SOCIETY. 31 JAN 2001, Bd. 123, Nr. 4, 31. Januar 2001 (2001-01-31), Seiten 709-721, XP002301113 ISSN: 0002-7863
- RONGSHI LI ET AL.: "Racemization of Vinylglycolate by Mandelates Racemase" JOURNAL OF ORGANIC CHEMISTRY, Bd. 60, Nr. 11, 1995, XP002301114 in der Anmeldung erwähnt

## Beschreibung

Die vorliegende Erfindung betrifft ein Verfahren zur mikrobiologischen Isomerisierung von alpha-Hydroxycarbonsäuren gemäß den Patentanspruchen.

## Stand der Technik:

Aus dem Stand der Technik bekannt sind sogenannte mikrobielle Mandelat Racemasen, welche in vitro oder in vivo in der Lage sind, Mandelsäure zu racemisieren (G.L. Kenyon, J.A. Gerlt, G.A. Petsko und J.W. Kozarich, "Mandelate Racemase: Structure-Function Studies of a Pseudosymmetric Enzyme", Accts. Chem. Res., 28, 178-186 (1995); R. Li, V.M. Powers, J.W. Kozarich und G.L. Kenyon, "Racemization of Vinylglycolate Catalyzed by Mandelate Racemase", J. Org. Chem., 60, 3347-3351 (1995); S.S. Schafer, A.T. Kallarakal, J.W. Kozarich, J.A. Gerlt, J.R. Clifton und G.L. Kenyon, "Mechanism of the Reaction Catalyzed by Mandelate Racemase: The Structure and Mechanistic Properties of the D270N Mutant", Biochemistry, 35, 5662-5669 (1996); B. Schnell et al., "Enzymatic Racemisation and its Application to Synthetic Biotranformations", Adv. Synth. Catal. 345, 653-666 (2003);U.T. Strauss and K. Faber, "Deracemization of (±) mandelic acid using a lipase-mandelate racemase two-enzyme system", Tetrahedron: Assymetry 10:4079-4081 (1999)).

Der Lactat-Metabolismus von Milchsäurebakterien ist beschrieben in S.-Q Liu, "Practical implications of lactat and pyruvate metabolism by lactic acid bacteria in food and beverage fermentations", Int. J. Food Microbiol. 83, 115-131 (2003).

Der nachveröffentlichte Poster-Abstract von S.M. Glück et al., "Lactate Racemase as a versatile tool for the racemazation of α-hydroxycarbolic acids", Chemicke Listy 97, 363, P121 (2003) erwähnt die Anwendung von Lactat Racemasen zur Racemisierung von alpha-hydroxysäuren.

Zugleich besteht ein großer Bedarf an der einfachen Herstellung chiraler, nicht-racemischer Substanzen, beispielsweise für die Wirkstoffproduktion in der pharmazeutischen Industrie. Die enzymkatalysierte Racemisierung einer stereoisomeren Form einer Verbindung unter Bildung des gewünschten Enantiomers würde einen geeigneten Weg zur Herstellung des gewünschten chiralen, nicht-racemischen Materials darstellen. Die bisher bekannten Racemasen sind jedoch durch hohe Substratspezifität gekennzeichnet. So eignet sich die Mandelat Racemase lediglich zur biokatalytischen Racemisierung von beta,gamma-ungesättigten alpha-Hydroxysäuren. Gesättigte alpha-Hydroxycarbonsäuren werden nicht akzeptiert (vgl. Felfer, U. et al., J. Mol. Catal. B: Enzymatic 15, 213 (2001)).

## Kurze Beschreibung der Erfindung:

Aufgabe der vorliegenden Erfindung ist die Bereitstellung von Mikroorganismen und Enzympräparaten, welche eine neuartige Racemase-Aktivität aufweisen, sowie die Bereitstellung von Verfahren zur Racemisierung stereoisomerer chemischer Verbindungen unter Verwendung dieser Enzyme bzw. Mikroorganismen.

Überraschenderweise wurde vorliegende Aufgabe gelöst durch Bereitstellung eines Verfahrens zur mikrobiologischen Isomerisierung von alpha-Hydroxycarbonsäuren nach Anspruch 1.

Ein erster Gegenstand der Erfindung betrifft ein Verfahren zur mikrobiologischen Isomerisierung von alpha-Hydroxycarbonsäuren der Formel I

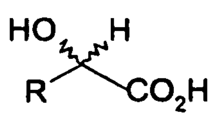

(I)

worin

R für geradkettiges oder verzweigtes $C_2$-$C_8$-Alkyl oder $C_2$-$C_8$-Alkenyl, vorzugsweise $C_2$-$C_8$ Alkyl, oder $-(CH_2)_n$-Cyc steht, worin n für einen ganzzahligen Wert von 0 bis 4, vorzugsweise 1, 2 oder 3, steht und Cyc für einen gegebenenfalls ein- oder mehrfach substituierten, ein- oder zweikernigen carbo- oder heterocyclischen Ring, wie z.B. einen gegebenenfalls substituierten aromatischen oder heteroaromatischen Ring, vorzugsweise einen gegebenenfalls substituierten einkernigen aromatischen Ring steht,

wobei man ein Substrat, enthaltend im wesentlichen eine erste stereoisomere Form ((R)- oder (S)-Form) einer alpha-Hydroxycarbonsäure der Formel (I), in der in Anspruch 1 definierten Weise isomerisiert und gegebenenfalls das dabei gebildete Isomerengemisch ((R)/(S)) oder ein gebildetes zweites Stereoisomer ((S) oder (R)-Form) isoliert, oder ein gebildetes zweites Stereoisomer aus dem Reaktionsgleichgewicht entfernt.

Vorzugsweise erfolgt die enzymatische Isomerisierung durch Umsetzung des Substrats mit einem enzymhaltigen Zellextrakt (z.B. Rohextrakt nach Zellaufschluss und Entfernung von Zellfragmenten) oder in Gegenwart ganzer Zellen, eines in Anspruch 1 definierten Mikroorganismus.

Insbesondere geeignete Mikroorganismen sind Bakterien der Gattung *Lactobacillus* und *Lactococcus.*

Gemäß einer bevorzugten Variante erfolgt die Umsetzung in Gegenwart ganzer Zellen von Mikroorganismen der Gattung *Lactobacillus,* oder ganzer Zellen eines rekombinanten Mikroorganismus, welche eine oben definierte Enzym-Aktivität exprimieren.

Bevorzugte Mikroorganismen sind ausgewählt unter *L. paracasei, L. delbrueckii, L. sakei* und *L. oris,* insbesondere unter den Stämmen *L. paracasei* DSM 20207 und DSM 2649, *L. delbrueckii* DSM20074, *L. sakei* DSM 20017 und *L. oris* DSM 4864.

Eine erfindungsgemäß brauchbare Enzymaktivität umfasst beispielsweise die Racemisierung wenigstens einer Verbindung ausgewählt unter der (R)- und/oder (S)-Form von Phenyllactat, 4-Fluorphenyllactat, 2-Hydroxy-4-phenylbuttersäure, 2-Hydroxy-4-methylpentancarbonsäure und 2-Hydroxy-3-methylbuttersäure.

In einer bevorzugten Ausführungsform des obigen Verfahrens wird aus dem gebildeten Isomerengemisch, vorzugsweise nach Isolierung des Gemischs aus dem Reaktionsmedium, z.B. durch Chromatographie, das gewünschte Stereoisomer im Wesentlichen entfernt, z.B. durch Chromatographie, chemische oder enzymatische stereoselektive Folgereaktion und gegebenenfalls weiterer Abtrennung des Folgeprodukts, und der Rückstand, der im wesentlichen das nicht-gewünschte Stereoisomer enthält, erneut einer Isomerisierung unterzogen.

Dies kann beliebig oft wiederholt werden, bis eine vollständige Umsetzung zum gewünschten Stereoisomer bzw. zum gewünschten Folgeprodukt davon, erreicht ist.

In einer weiteren bevorzugten Variante des obigen Verfahrens wird das gebildete Isomerengemisch, vorzugsweise nach Isolierung des Gemischs aus dem Reaktionsmedium, z.B. durch Chromatographie, einer chemischen oder enzymatischen stereoselektiven Folgereaktion unterzogen und das dabei anfallende Reaktionsgemisch, welches auch das nichtumgesetzte Isomer der Hydroxycarbonsäure enthält, erneut einer erfindungsgemäßen Isomerisierung unterzieht. Dies kann beliebig oft wiederholt werden, bis eine vollständige Umsetzung zum gewünschten Stereoisomer, bzw. zum gewünschten Folgeprodukt davon, erreicht ist.

In einer weiteren bevorzugten Variante des obigen Verfahrens, wird die Isomerisierungsreaktion mit einer chemischen oder enzymatischen, enantioselektiven Folgereaktion koppelt, vorzugsweise in einer sogenannten "Eintopfreaktion", wobei das gewünschte gebildete Stereoisomer der alpha-Hydroxycarbonsäure schrittweise oder kontinuierlich aus dem Reaktionsgleichgewicht entfernt wird.

Bevorzugte chemische oder enzymatische, enantioselektive Folgereaktionen zum erfindungsgemäßen Isomerisierungsschritt sind ausgewählt unter einer Veresterung und einer Amidierung einer stereoisomeren Form der alpha-Hydroxycarbonsäure. Die enantioselektive Folgereaktion kann insbesondere an einer Carboxyl- oder Hydroxylgruppe erfolgen.

Das erfindungsgemäße Verfahren dient auch als Screeningverfahren für Mikroorganismen welche oben definierte Enzym-Aktivität exprimieren, wobei man einen Mikroorganismus in welchem man diese Aktivität vermutet in Gegenwart eines Substrats, enthaltend im wesentlichen eine stereoisomere Form einer alpha-Hydroxycarbonsäure der obigen Formel I, kultiviert und das Reaktionsmedium auf Racemisierung (z.B. Abnahme der Menge der einen und/oder Zunahme der anderen stereoisomeren Form) des Substrats untersucht.

Ein solches Screeningverfahren ist nicht auf spezielle Mikroorgansimen beschränkt. Es kann grundsätzlich mit allen bekannten eu- und prokaryotischen Mikrorganismen, tierischen oder pflanzlichen Zellen durchgeführt werden. Bevorzugt sind aber solche Mikroorganismen, welche Lactat bilden oder metabolisieren, wie z.B. Milchsäure oder Propionibakterien. Insbesondere geeignete Quellen sind Bakterien der Gattung *Lactobacillus.*

Bevorzugt werden solche Mikroorganismen gescreent, welche das im wesentlichen stereoisomere Substrat zu 1 bis 100%, vorzugsweise zu 20 bis 100%, insbesondere 50 bis 100 % oder 80 bis 100% racemisieren (Razemisierung (%) = 2R/(R+S) x 100; R = Konzentration R-Form; S = Konzentration S-Form). Geeignete Umsätze liegen dabei im Bereich von 1 bis 50 %, vorzugsweise 5 bis 50%, insbesondere 15 bis 50 % oder 30 bis 50 % (Umsatz (%) = R/(R+S) x 100.

## Detaillierte Beschreibung der Erfindung:

### A. Allgemeine Begriffe und Definitionen

Werden keine andere Angaben gemacht, so gelten folgende allgemeine Bedeutungen:

"Racemate" stehen für äquimolare Mischungen der beiden Enantiomere einer optisch aktiven Verbindung.

"Racemisierung" bzw. "Isomerisierung" erfolgt erfindungsgemäß am alpha-Kohlenstoffatom der Hydroxycarbonsäure.

"Halogen" steht für Fluor, Chlor, Brom oder Jod, insbesondere Fluor oder Chlor.

"$C_2$-$C_8$-Alkyl" steht bevorzugt für geradkettige oder verzweigte Alkylreste mit 2 bis 8, insbesondere 2 bis 6 C-Atomen, wie Ethyl, i- oder n-Propyl, n-, i-, sec.- oder tert.-Butyl, n-Pentyl oder 2-Methyl-Butyl, n-Hexyl, 2-Methyl-pentyl, 3-Methyl-pentyl, 2-Ethyl-butyl.

"$C_2$-$C_8$-Alkenyl" steht für die ein- oder mehrfach, vorzugsweise einfach ungesättigten Analoga oben genannter Alkylreste mit 2 bis 8, insbesondere 2 bis 6 Kohlenstoffatomen, wobei die Doppelbindung in beliebiger Position der Kohlenstoffkette liegen kann.

"Aryl" steht für einen ein- oder mehrkernigen, vorzugsweise ein- oder zweikernigen, gegebenenfalls substituierten aromatischen Rest, insbesondere für Phenyl oder für ein über eine beliebige Ringposition gebundenes Naphthyl, wie 1- oder 2-Naphthyl. Diese Arylreste können gegebenenfalls 1 oder 2 gleiche oder verschiedene Substituenten tragen, ausgewählt unter Halogen, Niedrigalkyl, Niedrigalkoxy gemäß obiger Definition oder Trifluormethyl.

**B. Racemisierbare alpha-Hydroxycarbonsäuren**

Erfindungsgemäß durch Racemisierung umsetzbare alpha-Hydroxycarbonsäuren sind solche obiger Formel (I), worin R für geradkettiges oder verzweigtes Niedrigalkyl oder Niedrigalkenyl oder $-(CH_2)_n$-Cyc steht, worin n für einen ganzzahligen Wert von 0 bis 4 steht und Cyc für einen gegebenenfalls ein- oder mehrfach substituierten, ein- oder zweikernigen carbo- oder heterocyclischen Ring steht. Diese Verbindungen können in enantiomerenreiner Form, d.h. als R- oder S-Enantiomer oder als nicht-racemisches Gemisch der beiden Enantiomere erfindungsgemäß racemisiert werden.

Die zur enzymatischen Synthese verwendeten alpha-Hydroxycarbonsäuren der Formel I sind an sich bekannte Verbindungen und unter Anwendung allgemein bekannter organischer Syntheseverfahren zugänglich.

Als Beispiele für carbo- und heterocyclische Gruppen Cyc sind insbesondere ein- oder zweikernigen, vorzugsweise einkernige, Gruppen mit bis zu 4, wie z.B. 0, 1 oder 2 gleichen oder verschiedenen Ring-Heteroatomen, ausgewählt unter O, N und S sind zu nennen.

Diese carbo- oder heterocyclischen Ringe umfassen insbesondere 3 bis 12, vorzugsweise 4, 5 oder 6 Ring-Kohlenstoffatomen. Als Beispiele können genannt werden Cyclopropyl, Cyclobutyl, Cyclopentyl, Cyclohexyl, Cycloheptyl, die ein- oder mehrfach ungesättigten Analoga davon, wie Cyclobutenyl, Cyclopentenyl, Cyclopentadienyl, Cyclohexenyl, Cycloheptenyl, Cyclohexadienyl, Cycloheptadienyl, und Phenyl; sowie 5- bis 7-gliedrige gesättigte oder ein oder mehrfach ungesättigte heterocyclische Reste mit 1 bis 4 Heteroatomen, die ausgewählt sind unter O, N und S. Insbesondere sind zu nennen heterocyclische Reste, abgeleitet von Pyrrolidin, Tetrahydrofuran, Piperidin, Morpholin, Pyrrol, Furan, Thiophen, Pyrazol, Imidazol, Oxazol, Thiazol, Pyridin, Pyran, Pyrimidin, Pyridazin und Pyrazin.

Weiterhin sind zu nennen zweikernige Rests, bei welchen einer der oben erwähnten Carbo- oder Heterocyclen mit einem weiteren Heterocyclus oder Carbocyclus kondensiert ist, wie z.B. Reste abgeleitet von Cumaron, Indol, Chinolin und Naphthalin.

Die Reste Cyc können dabei über eine beliebige Ringposition, vorzugsweise über ein Ring-Kohlenstoffatom, gebunden sein.

Beispiele für geeignet Cyc-Reste sind Phenyl, Naphthyl, 2-Thienyl, 3-Thienyl; 2-Furanyl, 3-Furanyl; 2-Pyridyl, 3-Pyridyl oder 4-Pyridyl; 2-Thiazolyl, 4-Thiazolyl oder 5-Thiazolyl; 4-Methyl-2-thienyl, 3-Ethyl-2-thienyl, 2-Methyl-3-thienyl, 4-Propyl-3-thienyl, 5-n-Butyl-2-thienyl, 4-Methyl-3-thienyl, 3-Methyl-2-thienyl; 3-Chlor-2-thienyl, 4-Brom-3-thienyl, 2-lod-3-thienyl, 5-lod-3-thienyl, 4-Fluor-2-thienyl, 2-Brom-3-thienyl, und 4-Chlor-2-thienyl.

Die Reste Cyc können weiterhin ein- oder mehrfach, wie z.B. ein- oder zweifach, substituiert sein. Vorzugsweise sitzen die Substituenten an einem Ring-Kohlenstoffatom. Beispiele für geeignete Substituenten sind Halogen, Niedrigalkyl, Niedrigalkenyl, Niedrigalkoxy, -OH, -SH, $-NO_2$ oder $NR^2R^3$, wobei $R^2$ und $R^3$ unabhängig voneinander für H, Methyl oder Ethyl stehen. Bevorzugte Substituenten sind Halogenreste.

Eine weitere bevorzugte Gruppe von Cyc-Resten sind Aryl-Reste gemäß obiger Definition.

**C. Enzyme mit alpha-Hydroxycarbonsäure-Racemase-Aktivität** (nicht mehr Gegenstand der Erfindung)

Die erfindungsgemäßen Enzyme mit alpha-Hydroxycarbonsäure-Racemase-Aktivität sind insbesondere aus Mikroorganismen der Gattung *Lactobacillus* zugänglich.

Bevorzugte Enzyme sind aus den *Lactobacillus*-Stämmen *L. paracasei* DSM 20207 (DSM 15755) und DSM 2649 (DSM 15751), *L. delbrueckii* DSM20074 (DSM 15754), *L. sakei* DSM 20017 (DSM 15753 und *L. oris* DSM 4864 (DSM 15752) isolierbar. Diese sind bei der DSM (Deutsche Stammsammlung für Zellkulturen und Mikroorganismen) erhältlich.

Die Enzyme sind unter Anwendung üblicher präparativer biochemischer Methoden aus Zellkulturen isolierbar. Aus den isolierten Enzymen lassen sich dann in herkömmlicher Weise, z. B. durch Peptidfragmentierung und N-terminale Sequenzierung, erste Aminosäuresequenzinformationen ableiten.

Erfindungsgemäß mit umfasst sind ebenfalls "funktionale Äquivalente" der natürlichen, aus obigen Organsimen isolierbaren Enzyme mit alpha-Hydroxycarbonsäure-Racemase-Aktivität.

"Funktionale Äquivalente" oder Analoga der natürlichen Racemasen sind im Rahmen der vorliegenden Erfindung davon verschiedene Polypeptide, welche weiterhin die gewünschte biologische Aktivität, wie z.B. Substratspezifität, besitzen. So versteht man beispielsweise unter "funktionalen Äquivalenten" Enzyme, die wenigstens eine Verbindung ausgewählt unter Phenyllactat, 4-Fluorphenyllactat, 2-Hydroxy-4-phenylbuttersäure, 2-Hydroxy-4-methylpentancarbonsäure, 2-Hydroxy-3-methylbuttersäure racemisieren und die mindestens 20 %, bevorzugt mindestens 50 %, besonders bevorzugt mindestens 75 %, ganz besonders bevorzugt mindestens 90 % der Aktivität eines natürlichen Racemase-Enzyms aus einem der oben genannten Lactobacillus-Stämme oder eine höhere Aktivität als diese aufweisen.

Funktionale Äquivalente sind außerdem vorzugsweise von etwa pH 4 bis 10 stabil und besitzen vorteilhaft ein pH-Optimum bei etwa pH 5 bis 8 sowie ein Temperaturoptimum im Bereich von 20˚C bis 80˚C.

Unter "funktionalen Äquivalenten" versteht man erfindungsgemäß insbesondere auch Mutanten, welche in wenigstens einer Sequenzposition der natürlichen Aminosäuresequenzen eine andere als die natürliche Aminosäure aufweisen aber trotzdem eine der oben genannten biologischen Aktivitäten besitzen. "Funktionale Äquivalente" umfassen somit die durch eine oder mehrere Aminosäure-Additionen, -Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

"Funktionale Äquivalente" im obigen Sinne sind auch "Präkursoren" der beschriebenen Polypeptide sowie "funktionale Derivate" und "Salze" der Polypeptide.

"Präkursoren" sind dabei natürliche oder synthetische Vorstufen der Polypeptide mit oder ohne der gewünschten biologischen Aktiviät.

Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung.

"Funktionale Derivate" erfindungsgemäßer Enzyme können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der natürlichen Racemasesequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Erfindungsgemäß mit umfasste "funktionale Äquivalente" sind Homologe zu den natürlichen Proteinen. Diese besitzen wenigstens 60 %, vorzugsweise wenigstens 75% ins besondere wenigsten 85 %, wie z.B. 90%, 95% oder 99%, Homologie zu einer der natürlichen Aminosäuresequenzen, berechnet nach dem Algorithmus von Pearson und Lipman, Proc. Natl. Acad, Sci. (USA) 85(8), 1988, 2444-2448. Eine prozentuale Homologie eines erfindungsgemäßen homologen Polypeptids bedeutet insbesondere prozentuale Identität der Aminosäurereste bezogen auf die Gesamtlänge einer der Aminosäuresequenzen eines erfindungsgemäßen Enzyms oder einer Enzymuntereinheit.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

Homologe der erfindungsgemäßen Proteine oder Polypeptide können durch Mutagenese erzeugt werden, z.B.

durch Punktmutation oder Verkürzung des Proteins.

Homologe des erfindungsgemäßen Racemasen können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

**D. Kodierende Nukleinsäuresequenzen** (nicht mehr Gegenstand der Erfindung)

Gegenstand der Erfindung sind auch Nukleinsäuresequenzen, die für ein Enzym mit alpha-Hydroxycarbonsäure-Racemase-Aktivität kodieren. Bevorzugt sind Nukleinsäuresequenzen, umfassend die von den natürlichen Aminosäuresequenzen der aus obigen Mikroorganismen isolierbaren Racemasen abgeleiteten Nukleinsäuresequenzen.

Alle erfindungsgemäßen Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix, herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Gegenstand der Erfindung sind insbesondere Nukleinsäuresequenzen (einzel- und doppelsträngige DNA- und RNA-Sequenzen, wie z.B. cDNA und mRNA), kodierend für eines der erfindungsgemäßen Enzyme, und deren funktionalen Äquivalenten, welche z.B. unter Verwendung künstlicher Nukleotidanaloga zugänglich sind.

Die Erfindung betrifft sowohl isolierte Nukleinsäuremoleküle, welche für erfindungsgemäße Polypeptide bzw. Proteine oder biologisch aktive Abschnitte davon kodieren, als auch Nukleinsäurefragmente, die z.B. zur Verwendung als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem untranslatierte Sequenzen vom 3'- und/oder 5'-Ende des kodierenden Genbereichs enthalten

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologer Sequenzen in anderen Zelltypen und Organismen verwendbar sind. Solche Sonden bzw. Primer umfassen gewöhnlich einen Nukleotidsequenzbereich, der unter "stringenten" Bedingungen (siehe unten) an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der offenbarten Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Die erfindungsgemäßen Nukleinsäuresequenzen lassen sich prinzipiell aus allen Organismen identifizieren und isolieren. Vorteilhaft lassen sich die erfindungsgemäßen Nukleinsäuresequenzen aus Bakterien des obigen Typs isolieren.

Erfindungsgemäße Nukleinsäuresequenzen oder Derivate davon, Homologe oder Teile dieser Sequenzen, lassen sich beispielsweise mit üblichen Hybridisierungsverfahren oder der PCR-Technik aus anderen Mikroorganismen, z.B. über genomische oder cDNA-Banken, isolieren. Diese DNA-Sequenzen hybridisieren unter Standardbedingungen mit den erfindungsgemäßen Sequenzen. Zur Hybridisierung werden vorteilhaft kurze Oligonukleotide der konservierten Bereiche beispielsweise aus dem aktiven Zentrum, die über Vergleiche mit der alpha-Hydroxycarbonsäure-Racemase in dem Fachmann bekannter Weise ermittelt werden können, verwendet. Es können aber auch längere Fragmente der erfindungsgemäßen Nukleinsäuren oder die vollständigen Sequenzen für die Hybridisierung verwendet werden. Je nach der verwendeten Nukleinsäure (Oligonukleotid, längeres Fragment oder vollständige Sequenz) oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10 °C niedriger als die von DNA:RNA-Hybriden gleicher Länge.

Unter Standardbedingungen sind beispielsweise je nach Nukleinsäure Temperaturen zwischen 42 und 58 °C in einer wäßrigen Pufferlösung mit einer Konzentration von 0,1 bis 5 x SSC (1 X SSC = 0,15 M NaCl, 15 mM Natriumcitrat, pH 7,2) oder zusätzlich in Gegenwart von 50% Formamid wie beispielsweise 42 °C in 5 x SSC, 50% Formamid zu verstehen. Vorteilhafterweise liegen die Hybridisierungsbedingungen für DNA:DNA-Hybride bei 0,1 x SSC und Temperaturen von etwa 20 °C bis 45 °C, bevorzugt von etwa 30 °C bis 45 °C. Für DNA:RNA-Hybride liegen die Hybridisierungsbedingungen vorteilhaft bei 0,1 x SSC und Temperaturen von etwa 30 °C bis 55 °C, bevorzugt von etwa 45 °C bis 55 °C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik, wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Harnes and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Gegenstand der Erfindung sind auch Derivate der konkret offenbarten oder ableitbaren Nukleinsäuresequenzen.

So können weitere erfindungsgemäße Nukleinsäuresequenzen von den natürlichen Sequenzen abgeleitet sein und sich davon durch Addition, Substitution, Insertion oder Deletion einzelner oder mehrerer Nukleotide unterscheiden, aber weiterhin für Polypeptide mit dem gewünschten Eigenschaftsprofil kodieren.

Erfindungsgemäß umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Gegenstand sind ebenso durch konservative Nukleotidsubstutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Unter Derivaten der erfindungsgemäßen Nukleinsäuren sind beispielsweise Allelvarianten zu verstehen, die mindestens 40 % Homologie auf der abgeleiteten Aminosäureebene, bevorzugt mindestens 60 % Homologie, ganz

besonders bevorzugt mindestens 80 % Homologie über den gesamten Sequenzbereich aufweisen (bezüglich Homologie auf Aminosäureebene sei auf obige Ausführungen zu den Polypeptiden verwiesen). Über Teilbereiche der Sequenzen können die Homologien vorteilhaft höher liegen.

Weiterhin sind unter Derivaten auch Homologe der erfindungsgemäßen Nukleinsäuresequenzen, beispielsweise pilzliche oder bakterielle Homologe, verkürzte Sequenzen, Einzelstrang-DNA oder RNA der kodierenden und nichtkodierenden DNA-Sequenz, zu verstehen. So besitzten z.B. Homologe zur der SEQ ID NO: 1 auf DNA-Ebene eine Homologie von mindestens 40 %, bevorzugt von mindestens 60 %, besonders bevorzugt von mindestens 70 %, ganz besonders bevorzugt von mindestens 80 % über den gesamten in SEQ ID NO: 1 angegebenen DNA-Bereich.

Außerdem sind unter Derivaten beispielsweise Fusionen mit Promotoren zu verstehen. Die Promotoren, die den angegebenen Nukleotidsequenzen vorgeschalten sind, können durch ein oder mehrere Nukleotidaustausche, Insertionen, Inversionen und/oder Deletionen verändert sein, ohne dass aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Unter Derivaten sind auch Varianten zu verstehen, deren Nukleotidsequenz im Bereich von - 1 bis -1000 Basen stromaufwärts vor dem Startkodon oder 0 bis 1000 Basen stromabwärts nach dem Stopkodon so verändert wurde, dass die Genexpression und/oder die Proteinexpression verändert, bevorzugt erhöht wird.

Weiterhin umfasst die Erfindung auch Nukleinsäuresequenzen, welchen mit oben genannten kodierenden Sequenzen unter "stingenten Bedingungen" hybridisieren. Diese Polynukleotide lassen sich bei der Durchmusterung von genomischen oder cDNA-Banken auffinden und gegebenenfalls daraus mit geeigneten Primern mittels PCR vermehren und anschließend beispielsweise mit geeigneten Sonden isolieren. Darüber hinaus können erfindungsgemäße Polynukleotide auch auf chemischem Wege synthetisiert werden. Unter dieser Eigenschaft versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, vorzugsweise zu 90-100%, komplementär sein. Die Eigenschaft komplementärer Sequenzen, spezifisch aneinander binden zu können, macht man sich beispielsweise in der Northern- oder Southem-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northem-Blot-Technik die Verwendung einer 50 - 70 ˚C, vorzugsweise 60 - 65 ˚C warmen Waschlösung, beispielsweise 0,1x SSC-Puffer mit 0,1% SDS (20x SSC: 3M NaCl, 0,3M Na-Citrat, pH 7,0) zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z:B. in Ausubel et al., Current Protocols in Molecular Biology, John Wiley & Sons, N.Y. (1989), 6.3.1-6.3.6. beschrieben.

**E. Konstrukte** (nicht mehr Gegenstand der Erfindung)

Gegenstand der Erfindung sind außerdem Expressionskonstrukte, enthaltend unter der genetischen Kontrolle regulativer Nukleinsäuresequenzen eine für ein erfindungsgemäßes Enzym kodierende Nukleinsäuresequenz; sowie Vektoren, umfassend wenigstens eines dieser Expressionskonstrukte.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz.

Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Unter einem erfindungsgemäßen Nukleinsäurekonstrukt sind insbesondere die natürlichen alpha-Hydroxycarbonsäure-Racemasegene und die Derivate und Homologen davon zu verstehen, die mit einem oder mehreren Regulationssignalen vorteilhafterweise zur Steuerung, z.B. Erhöhung, der Genexpression operativ oder funktionell verknüpft wurden.

Zusätzlich zu diesen Regulationssequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die kodierende Sequenz insertiert und der natürliche Promotor mit seiner Regulation wurde nicht entfernt. Stattdessen wird die natürliche Regulationssequenz so mutiert, dass keine Regulation mehr erfolgt und die Genexpression gesteigert wird.

Ein bevorzugtes Nukleinsäurekonstrukt enthält vorteilhafterweise auch eine oder mehrere der schon erwähnten "Enhancer" Sequenzen, funktionell verknüpft mit dem Promotor, die eine erhöhte Expression der Nukleinsäuresequenz ermöglichen. Auch am 3'-Ende der DNA-Sequenzen können zusätzliche vorteilhafte Sequenzen inseriert werden, wie weitere regulatorische Elemente oder Terminatoren. Die erfindungsgemäßen Nukleinsäuren können in einer oder mehreren Kopien im Konstrukt enthalten sein. Im Konstrukt können noch weitere Marker, wie Antibiotikaresistenzen oder Auxotrophien komplementierende Gene, gegebenenfalls zur Selektion auf das Konstrukt enthalten sein.

Vorteilhafte Regulationssequenzen für das erfindungsgemäße Verfahren sind beispielsweise in Promotoren wie cos-, tac-, trp-, tet-, trp-tet-, Ipp-, lac-, Ipp-lac-, $lacI^{q-}$, T7-, T5-, T3-, gal-, trc-, ara-, rhaP ($rhaP_{BAD}$)SP6-, lambda-$P_R$- oder im lambda-$P_L$-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFalpha , AC, P-60, CYC1, GAPDH, TEF, rp28, ADH enthalten. Es können auch künstliche Promotoren für die Regulation verwendet werden.

Das Nukleinsäurekonstrukt wird zur Expression in einem Wirtsorganismus vorteilhafterweise in einen Vektor, wie beispielsweise einem Plasmid oder einem Phagen inseriert, der eine optimale Expression der Gene im Wirt ermöglicht. Unter Vektoren sind außer Plasmiden und Phagen auch alle anderen dem Fachmann bekannten Vektoren, also z.B. Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden. Diese Vektoren stellen eine weitere Ausgestaltung der Erfindung dar. Geeignete Plasmide sind beispielsweise in E. coli pLG338, pACYC184, pBR322, pUC18, pUC19, pKC30, pRep4, pHS1, pKK223-3, pDHE19.2, pHS2, pPLc236, pMBL24, pLG200, pUR290, $pIN\text{-}III^{113}$-B1,$\lambda$gt11 oder pBdCI, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2alphaM, pAG-1, YEp6, YEp13 oder pEMBLYe23 oder in Pflanzen pLGV23, $pGHIac^+$, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

Vorteilhafterweise enthält das Nukleinsäurekonstrukt zur Expression der weiteren enthaltenen Gene zusätzlich noch 3'- und/oder 5'-terminale regulatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der erfindungsgemäßen Nukleinsäure bestehen.

Für eine optimale Expression heterologer Gene in Organismen ist es vorteilhaft die Nukleinsäuresequenzen entsprechend des im Organismus verwendeten spezifischen "codon usage" zu verändern. Der "codon usage" läßt sich anhand von Computerauswertungen anderer, bekannter Gene des betreffenden Organismus leicht ermitteln.

Die Herstellung einer erfindungsgemäßen Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden.

**F. Erfindungsgemäß brauchbare Wirte**

Mit Hilfe der Vektoren sind rekombinante Mikroorganismen herstellbar, welche beispielsweise mit wenigstens einem Vektor transformiert sind und zur Produktion der erfindungsgemäß branchbare Enzyme eingesetzt werden können. Vorteilhafterweise werden die oben beschriebenen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Erfindungsgemäß sind auch homolog rekombinierte Mikroorganismen herstellbar. Dazu wird ein Vektor hergestellt, der zumindest einen Abschnitt eines erfindungsgemäßen Gens oder einer kodierenden Sequenz enthält, worin gegebenenfalls wenigstens eine Aminosäure-Deletion, -Addition oder -Substitution eingebracht worden ist, um die erfindungsgemäße Sequenz zu verändern, z.B. funktionell zu disrumpieren ("Knockour-Vektor). Die eingebrachte Sequenz kann z.B. auch ein Homologes aus einem verwandten Mikroorganismus sein oder aus einer Säugetier-, Hefe- oder Insektenquelle abgeleitet sein. Der zur homologen Rekombination verwendete Vektor kann alternativ derart ausgestaltet sein, daß das endogene Gen bei homologer Rekombination mutiert oder anderweitig verändert ist, jedoch noch das funktionelle Protein kodiert (z.B. kann der stromaufwärts gelegene regulatorische Bereich derart verändert sein, dass dadurch die Expression des endogenen Proteins verändert wird). Der veränderte Abschnitt des erfindungsgemäßen Gens ist im homologen Rekombinationsvektor. Die Konstruktion geeigneter Vektoren zur homologen Rekombination ist z.B. beschrieben in Thomas, K.R. und Capecchi, M.R. (1987) Cell 51:503.

Als rekombinante Wirtsorganismen für die Nukleinsäure oder das Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden gram-positive oder gram-negative Bakterien verwendet. Ganz besonders bevorzugt ist die Gattung und Art Escherichia coli.

Der Wirtsorganismus oder die Wirtsorganismen gemäß der Erfindung enthalten dabei vorzugsweise mindestens eine Nukleinsäuresequenz, Nukleinsäurekonstrukte oder Vektoren, die für ein Enzym mit hierin definierter Aktivität kodieren.

Die im erfindungsgemäßen Verfahren verwendeten Organismen werden je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen von 0 °C bis 100 °C, bevorzugt von 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann "batch"-weise, "semi batch"-weise oder kontinuierlich erfolgen. Nährstoffe können zu beginn der Fermentation vorgelegt oder semikontinuierlich oder kontinuierlich nachgefüttert werden. Das Keton kann direkt zur Anzucht gegeben werden oder vorteilhaft nach Anzucht. Die Enzyme können nach dem in den Beispielen beschriebenen Verfahren aus den Organismen isoliert werden oder als Rohextrakt für die Reaktion verwendet werden.

**G. Rekombinante Herstellung der erfindungsgemäßen Enzyme:** (nicht mehr Gegenstand der Erfindung)

Gegenstand der Erfindung sind weiterhin Verfahren zur rekombinanten Herstellung erfindungsgemäße Polypeptide oder funktioneller, biologisch aktiver Fragmente davon, wobei man einen Polypeptide-produzierenden Mikroorganismus kultiviert, gegebenenfalls die Expression der Polypeptide induziert und diese aus der Kultur isoliert. Die Polypeptide können so auch in großtechnischem Maßstab produziert werden, falls dies erwünscht ist.

Der rekombinante Mikroorganismus kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Polypeptide nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie

Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Vorteilhaft kann es sein, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig können diese Anker auch zur Erkennung der Proteine verwendet werden. Zur Erkennung der Proteine können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Proteine verwendet werden.

**H. NIchtrekombinante Isolierung erfindungsgemäßer Enzyme** (nicht mehr Gegenstand der Erfindung)

Die Isolierung erfindungsgemäßer Enzyme erfolgt zweckmäßigerweise aus einer der oben beschriebenen natürlichen Quellen (*Lactobacillus*-Stämme) bzw. aus einem anderen, mit Hilfe eines erfindungsgemäßen Screeningverfahrens identifizierten, alpha-Hydroxycarbonsäure-Racemase-Aktivität exprimierenden Mikroorganismus.

Der Mikroorganismus kann nach bekannten Verfahren kultiviert und fermentiert werden. Die Zellen werden dann, falls die Polypeptide nicht in das Kulturmedium sezemiert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Polypeptide kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

**I. Durchführung des erfindungsgemäßen Verfahrens zur Isomerisierung von alpha-hydroxycarbonsäuren**

Das erfindungsgemäße Verfahren wird vorteilhaft bei einer Temperatur im Bereich von 0 ˚C bis 95 ˚C, bevorzugt von 10 ˚C bis 85 ˚C, besonders bevorzugt von 15 ˚C bis 75 ˚C, insbesondere 25 bis 40 ˚C , durchgeführt.

Der pH-Wert im erfindungsgemäßen Verfahren wird vorteilhaft in einem Bereich von pH 4 bis 12, bevorzugt von pH 4,5 bis 9, besonders bevorzugt von pH 5 bis 8 gehalten.

Für das erfindungsgemäße Verfahren können wachsende Zellen verwendet werden, die die erfindungsgemäßen Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Werden für das erfindungsgemäße Verfahren freie Organismen oder immobilisierte Enzyme verwendet, so werden diese vor der Extraktion zweckmäßigerweise abgetrennt, beispielsweise über eine Filtration oder Zentrifugation.

Das im erfindungsgemäßen Verfahren hergestellte Produkt lässt sich vorteilhaft aus der wässrigen Reaktionslösung über Extraktion oder Destillation gewinnen. Die Extraktion kann zur Erhöhung der Ausbeute mehrfach wiederholt werden. Beispiele für geeignete Extraktionsmittel sind übliche, dem Fachmann geläufige Lösungsmittel, wie Toluol, Methylenchlorid, Diisopropylether, Benzol, MTBE oder Essigester, ohne darauf beschränkt zu sein.

Nach Einengen der organischen Phase können die Produkte in der Regel in guten chemischen Reinheiten, das heißt größer 80 % chemische Reinheit, gewonnen werden. Nach Extraktion kann die organische Phase mit dem

Produkt aber auch nur zum Teil eingeengt werden und das Produkt auskristallisiert werden. Dazu wird die Lösung vorteilhaft auf eine Temperatur von 0 ˚C bis 10 ˚C abgekühlt. Die Kristallisation kann auch direkt aus der organischen Lösung oder aus einer wässrigen Lösung erfolgen. Das auskristallisierte Produkt kann nochmals im gleichen oder in einem anderen Lösungsmittel zur erneuten Kristallisation aufgenommen werden und nochmals kristallisiert werden.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

**K. Weitere Anwendungen der Erfindung**

Die vorliegende Erfindung eröffnet zahlreiche weitere Anwendungsmöglichkeiten der erfindungsgemäßen Isomerisierungsreaktionen. Ohne darauf beschränkt zu sein, seien folgende Beispiele genannt:

a) Racemisierung von alpha-Hydroxycarbonsäuren zur "Rückführung" des unerwünschten Stereoisomers in klassischen Racemattrennungen nach Abtrennung vom gewünschten Produkt. Der Vorteil liegt in der sauberen enzymatischen Racemisierungs-Reaktion; chemisch katalysierte Racemisierungen verlaufen dagegen meist unter drastischen Reaktionsbedingungen, die zu Zersetzung, zur Bildung von Nebenprodukten und zu Seitenreaktionen (Eliminierung, etc.) führen. (Vgl. auch: Kontrollierte Racemisierung von organischen Verbindungen: E. J. Ebbers, G. J. A. Ariaans, J. P. M. Houbiers, A. Bruggink, B. Zwanenburg, Tetrahedron, 1997, 53, 9417-9476)

b) Stufenweise Deracemisierung von alpha-Hydroxycarbonsäuren. Sofern die oben definierte Enzym aktivität (z.B. aufgrund der benötigten Reaktionsbedingungen) nicht direkt mit einem (chemisch oder enzymatisch katalysierten) enantioselektiven Schritt im "Eintopfverfahren" eingesetzt werden kann, wird die enzymatische Racemisierung nach dem enantioselektiven Schritt ohne Trennung der enantiomeren Produkte durchgeführt. (vgl. analoges Beispiel unter Verwendung von Mandelat-Racemase: U. T. Strauss, K. Faber, Tetrahedron: Asymmetry, 1999, 10, 4079-4081).

c) Der ideale Fall einer Anwendung entspricht einer direkten Kombination der oben definierten Enzym aktivität mit einem chemo- oder biokatalytischen Schritt im "Eintopfverfahren", einer sogenannten "Dynamischen Racemattrennung". Solche Verfahren sind elegant und extrem effektiv.

Als Beispiel für einen enantioselektiven Schritt, der mit einer erfindungsgemäßen Razemisierung kombinierbar ist, wären zu nennen:

(i) Lipase-katalysierte Veresterung, bzw. Acyl-Transfer-Reaktion (U. T. Strauss, K. Faber, Tetrahedron: Asymmetry, 1999, 10, 4079-4081).

(ii) Lipase- oder Protease-katalysierte Amidbildung. (F. van Rantwijk, M. A. P. J. Hacking, R. A. Sheldon, Monatsh. Chem. /Chem. Monthly, 2000, 131, 549-569.)

Die obige Beschreibung und die nachstehenden Beispiele dienen nur der Verdeutlichung der Erfindung. Die für den Fachmann offensichtlichen, zahlreich möglichen Abwandlungen sind erfindungsgemäß ebenfalls umfasst.

## Experimenteller Teil

### A. Allgemeine Angaben

#### 1. Verwendete Geräte und Methoden

Dünnschichtchromatographie

Die Umsatzkontrolle der Reaktionen sowie die Reinheitsprüfung der Produkte erfolgte mittels Dünnschichtchromatographie. Zu diesem Zweck wurde Kieselgel $60_{F254}$ auf Alufolie von Merck verwendet. Die Detektion erfolgte sowohl durch UV-Licht (254 mm) als auch durch Besprühen mit Molybdatlösung [$(NH_4)_6Mo_7O_{24}$ x $4H_2O$ (100g/l) und $Ce(SO_4)_2$ x $4H_2O$ (4g/l) in $H_2SO_4$ (10%)] und anschließendem Erhitzen.

Säulenchromatographie

Die Reinigung der Razemisierungprodukte erfolgte säulenchromatographisch. Dazu wurde Kieselgel der Korngröße 43-63μm von der Firma Merck als stationäre Phase verwendet. Als Eluationsmittel dienten Gemische aus Benzin (B) und Essigsäureethylester (EE), wobei die Zusammensetzung dem jeweiligen Trennungsproblem angepasst wurde.

Die Trennung erfolgte unter erhöhtem Druck.

Gaschromatographie

Die gaschromatigraphische Trennung der Razemisierungsprodukte wurde mit einem Varian Gaschromotographen 3800 - Split/Splitless Injektor (FID) - durchgeführt. Als Säule diente eine Chirasil-DEX, Chrompack, Permethyl-β-cyclodextrin, chemisch gebunden, 25m x 0.32mm x 0.25 µm, $H_2$. Beispiele geeigneter Trennbedingungen sind in folgender Tabelle 1 angegeben.

Tabelle 1: Trennung von Enantiomeren am chiralen GC

| Verbindung | Säulenvordruck [psi] | Temperaturprogramm [˚C] | Retentionszeit [min] |
|---|---|---|---|
| Lactat | 12 | 45˚C | 4,6 (*R*)<br>6,0 (*S*) |
| Phenyllactat | 12 | 125˚C | 7,5 (*R*)<br>8,5 (*S*) |
| 4-Fluorphenyllactat | 12 | 80˚C/0-5˚C/min<br>170˚C | 12,0 (*R*)<br>12,5 (*S*) |

NMR

$^1H$- und $^{13}C$-NMR-Spektren wurden mit einem Bruker 360- bzw. 500 MHz aufgenommen, die chemischen Verschiebungen sind in ppm (δ-Skala) angegeben, als interner Standard wurde Tetramethylsilan (TMS) verwendet.

HPLC

Die Bestimmungen des e.e. Werts erfolgten über ein HPLC-System von JASCO mit Pumpen vom Typ PU-980 und einem UV-Detektor MD 910. Für die Trennung wurde eine DAICEL Chiralpack AD (0.46 cm x 25 cm) Säule verwendet.

Schmelzpunkte

Die Schmelzpunkte wurden mit einem MPD 350.BML.5 der Firma GALLENKAMP bestimmt.

**2. Mikrobiologische Methoden und Materialien**

2.1 Anzucht der Kulturen

Bakterienstämme

Tabelle 2 enthält eine Aufstellung der untersuchten Stämme, deren Aufzuchtbedingungen und Hinterlegungsnummern.

Tabelle 2: Wachstumsbedingungen untersuchter Bakterienstämme

| **Stamm** | **DSM Nummer** | **Medium** | **Temperatur** |
|---|---|---|---|
| *L. haloferax volcanii* | 5176 | 372 | 37˚C |
| *L. haloarcula vallismortis* | 3756 | 372 | 37˚C |
| *L. paracasei* | 20008 | 11 | 30˚C |
| *L. paracasei* | 20207 | 11 | 30˚C |
| *L. paracase* | 2649 | 11 | 30˚C |
| *L. acidophilus* | 20079 | 11 | 30˚C |
| *L. brevis* | 20054 | 11 | 30˚C |

(fortgesetzt)

| Stamm | DSM Nummer | Medium | Temperatur |
|---|---|---|---|
| *L. piscicola* | 20722 | 92 | 30˚C |
| *L. halotolerans* | 20190 | 11 | 30˚C |
| *L. confusus* | 20196 | 11 | 30˚C |
| *L. acetotolerans* | 20749 | 231 | 30˚C |
| *L. delbrueckii* ssp. *delbrueckii* | 20074 | 11 | 37˚C |
| *L. kandleri* | 20593 | 11 | 30˚C |
| *L. fructosus* | 20349 | 11 | 30˚C |
| *L. farciminis* | 20184 | 11 | 30˚C |
| *L. gasseri* | 20243 | 11 | 37˚C |
| *L. alimentarius* | 20249 | 11 | 30˚C |
| *L. jensenii* | 20557 | 11 | 37˚C |
| *L. curvatus* | 20010 | 11 | 30˚C |
| *L. sakei* ssp.*sakei* | 20017 | 11 | 30˚C |
| *L. oris* | 4864 | 11 | 37˚C |

Stammhaltung

Die Stammhaltung erfolgte durch Einfrieren der Zellen in speziellen Gefrierlösungen. Dazu wurde die Kultur in sterile Zentrifugenbecher überführt, 30 min bei 8000 rpm und 4˚C zentrifugiert und die überstehende Lösung abdekantiert. Anschließend wurden die Zellen in Gefrierlösung aufgeschlemmt, in sterile Vials überführt und bei -70˚C aufbewahrt. Als Gefrier-Lösung wurde entweder eine Lösung von 20 Vol.-% DMSO und 0,7Gew.-% NaCl in $H_2O$ (Fa. Sölkner) oder Glycerin mit 5 Vol.-%, $H_2O$ (Fa. Weigers) verwendet.

Anzucht der Stämme

Das Wachstum der Kulturen erfolgte in 1000 ml Erlenmeyerkolben im Trockenschrank bei 30˚C bzw. 37˚C (je nach Bakterienstamm). Zunächst wurde für jeden Bakterienstamm jeweils 1 Liter des betreffenden sterilen Mediums auf vier Kolben aufgeteilt und mit je 500 μl der zuvor aufgetauten Zelllösung beimpft. Alle sterilen Arbeitsschritte wurden im Laminarflow (Heraeus "Herasafe", Typ HS9) durchgeführt.

Ernte der Zellen

Nach einer Wachstumsphase von 3 bis 15 Tagen, je nach Bakterienstamm, wurden die Zellen geerntet. Dazu wurde die Kultur in Zentrifugenbecher überführt, 20 min bei 8000 rpm und 4˚C zentrifugiert und der Überstand abdekantiert. Zum Waschen wurden die Zellen mit etwa 40 ml Puffer (50mM Bis-Tris, 0,01 M $MgCl_2$, pH = 6) aufgeschlemmt und wiederum 20 min zentrifugiert. Der Waschvorgang wurde zweimal durchgeführt. Anschließend wurden die Zellen erneut mit wenig Puffer aufgeschlemmt, in einem Rundkolben überführt und schockgefroren. Dies erfolgte durch Schwenken des Kolbens in einem mit flüssigem Stickstoff gefüllten Dewar-Gefäß. Die Gefriertrocknung wurde mit einem Lyophilisator der Firma Braun ("Christ Alpha 1-4") durchgeführt. Die lyophilisierten Zellen wurden in Glasflaschen gefüllt und bis zur ihrer Verwendung im Kühlschrank aufbewahrt.

2.2 Nährmedien und Sterilisation

Zur Anzucht der Mikroorganismen wurden die für die jeweiligen Bakterienstämme empfohlenen Nährmedien verwendet. Diese wurden vor dem Beimpfen durch Autoklavieren (Varioklav-Dampfsterilisator, H+P Labortechnik GmbH, München) bei 121˚C und 1 bar Überdruck sterilisiert.

Medium 11

Medium 11 wurde als Nährmedium für folgende Bakterienstämme verwendet: *L. paracasei, L. acidophilus, L. brevis, L. halotolerans, L, confusus, L. farciminis, L. gasseri, L. alimentarius, L. jensenii, L. delbrueckii, L. curvatus, L. sakei ssp. sakei, L. kandeleri und L. fructosus.*

In Tabelle 3 ist die Zusammensetzung des Mediums angegeben. Um eine mögliche Maillard-Reaktion sowie ein Ausfallen der Salze zu verhindern, wurden die Medienbestandteile getrennt sterilisiert und erst nach der Sterilisation vereinigt. Die Maillard-Reaktion tritt auf, wenn Verbindungen wie reduzierende Zucker mit Aminosäuren bzw. Proteinen reagieren, und verursacht eine dunkle Färbung des Mediums.

Tabelle 3: Zusammensetzung des Mediums 11

| **Substanz** | **Konzentration [g/l]** |
|---|---|
| Casein Pepton (Fa. Oxoid) | 10,00 |
| Pepton (Fa. Oxoid) | 10,00 |
| Hefeextrakt (Fa. Oxoid) | 5,00 |
| Glukose | 20,00 |
| $K_2HPO_4$ | 2,00 |
| Natriumacetat Trihydrat | 8,00 |
| Ammoniumcitrat | 2,00 |
| $MgSO_4$ x 7 $H_2O$ | 0,20 |
| $MnSO_4$ x $H_2O$ | 0,05 |
| Tween 80 | 1,00 |

Medium 231

Für *L. acetotolerans* wurde Medium 231 verwendet, welches sich nur im pH-Wert von Medium 11 unterscheidet. Die in Tabelle 3 angeführten Bestandteile wurden bereits vor dem Sterilisieren vereinigt und der pH auf 5.2 eingestellt.

Medium 92

Medium 92 wurde für *L. piscicola* verwendet. Die Zusammensetzung ist in Tabelle 4 angegeben.

Tabelle 4: Zusammensetzung des Mediums 92

| **Substanz** | **Konzentration [g/l]** |
|---|---|
| Sojaextrakt tryptisch aufgeschlossen (Fa. Oxoid) | 30,00 |
| Hefeextrakt (Fa. Oxoid) | 3,00 |

Medium 372

Medium 372 wurde für die Stämme *Haloferax volcanii* und *Haloarcula vallismortis* verwendet.

Tabelle 5: Zusammensetzung des Medium 372

| **Substanz** | **Konzentration [g/l]** |
|---|---|
| Hefeextrakt (Fa. Oxoid) | 5,00 |
| Casaminosäuren (Fa. Oxoid) | 5,00 |
| Natriumglutamat (Fa. Oxoid) | 1,00 |
| KCl | 2,00 |
| Natriumcitrat | 3,00 |

(fortgesetzt)

| Substanz | Konzentration [g/l] |
|---|---|
| $MgSO_4 \times 7\ H_2O$ | 20,00 |
| NaCl | 200,00 |
| $FeCl_2 \times 4\ H_2O$ | 0,036 |
| $MnCl_2 \times 4\ H_2O$ | 0,00036 |

B. Durchgeführte Versuche

**Beispiel 1: Synthese von Substraten und Referenzmaterial**

a) Darstellung von enantiomerenreinem 2-Hydroxy-3-phenylpropionat

Ansatz: 1g enantiomerenreines Phenylalanin (6,05 mmol) wird in 12 ml $H_2SO_4$ (1M) gelöst. Unter Eiskühlung werden 1,66 g (24 mmol) $NaNO_2$ portionsweise zugegeben. Nach der Zugabe wird die Kühlung entfernt und der Ansatz bei Raumtemperatur über Nacht weitergerührt.

Aufarbeitung: Die wässrige Lösung wird dreimal mit je 4 ml Ethylether extrahiert, die organische Phase wird mit gesättigter Kochsalzlösung gewaschen und die Kochsalzlösung einmal mit Ethylether rückextrahiert. Die gesammelte organische Phase wird über $Na_2SO_4$ getrocknet und das Lösungsmittel mit Rotavapor entfernt. Der gelbe, ölige Rückstand wird mit 2 ml Hexan versetzt und durch Ankratzen mit einem Glasstab zur Kristallisation gebracht. Es bildet sich ein Niederschlag von weißen Kristallen. Die Kristalle werden dreimal mit etwa 2 ml Hexan gewaschen und das restliche Lösungsmittel abrotiert.

Drehwerte (Literatur): *(S)*-Phenyllactat: $[\alpha]=\ _D^{25}$ = -20,8 (*c* = 2, $H_2O$)
*(R)*-Phenyllactat: $[\alpha]=\ _D^{25}$ = +20,9 (*c* = 2,3 $H_2O$)

b) Darstellung von *rac*-4-Fluor-phenyllactat *rac*-3

Ansatz: 400 mg (2.2 mmol) rac-4-Fluor-phenylalanin, 670 mg (9,7 mmol) $NaNO_2$ in 4,8 ml $H_2SO$ (1M).
Ausbeute: 197 mg (49.0 %), weiße Kristalle
$^1$H-NMR: (360 MHz, $CDCl_3$) $\delta$ = 2,97 (1H, dd, $J_1$ = 7,2 Hz, $J_2$ = 14,4 Hz, $CH_2$); 3,17 (1H, dd, $J_1$ = 3,6 Hz, $J_2$ = 14,4 Hz, $CH_2$); 4,49 (1H,dd, $J_1$ = 3,6 Hz, $J_2$ = 6,1 Hz, CH-OH); 6,98 - 7,02 (2H, m, Ar); 7,20 - 7,28 (2H, m, Ar).
$^{13}$C-NMR: (90 MHz, $CDCl_3$) $\delta$ = 39,2 ($CH_2$); 70,9 (CH-OH); 115,3 (d, J = 21,6 Hz, Ar-m); 130,9 (d, J = 21,6 Hz, Ar-o); 131,6 (d, J = 3,6 Hz, Ar-i); 162,1 (d, J = 243,9 Hz, Ar-p); 178,1 (COOH).
DC-Daten: LM:EE; $R_F$ = 0,35
Schmelzpunkt: 73˚C

c) Darstellung von *(S)*-4-Fluor-phenyllactat *(S)*-3

Ansatz: 452 mg (2,5 mmol) *(S)*-4-Fluor-phenylalanin, 750 mg (10,9 mmol) $NaNO_2$ in 5,4 ml $H_2SO_4$ (1 M).
Ausbeute: 330 mg (72,5 %), weiße Kristalle
$^1$H-NMR: siehe rac-4-Fluor-phenyllactat
$^{13}$C-NMR: siehe rac-4-Fluor-phenyllactat
DC-Daten: LM:EE, $R_F$ = 0,35
Schmelzpunkt 69˚C

Die Synthese der Verbindungen
(*S*)-(+)-2-Hydroxy-3-methylbutansäue
(*S*)-(-)-2-Hydroxy-4-methylpentansäure
(*S*)-2-Hydroxy-4-phenylbuttersäure
(*S*)-2-Hydroxy-3-phenylpropansäure
erfolgte in analoger Weise zu Beispiel 1 ausgehend von den korrespondierenden, käuflich erhältlichen enantiomerenreinen Aminosäuren.

**Beispiel 2: Screening nach Racemase-Aktivität**

Um einen ersten Anhaltspunkt bezüglich der Racemisierung-Aktivität der Mikroorganismen zu erhalten, wurde

untersucht, ob diese eine Racemisierung des (natürlichen) Substrats *(S)*-Lactat 1 und/oder der (nicht-natürlichen) Substrate *(S)*-Phenyllactat 2 sowie (S)-4-Fluorphenyllactat 3 zeigen.

Für das Screening wurden ca. 10 mg lyophilisierte Zellen in einer Eppendorffiole in 900 µl Bis-Tris-Puffer (50 mM Bis-Tris, pH = 6) eine Stunde bei 42°C und 150 rpm rehydratisiert. Anschließend erfolgte die Zugabe von je 10 mg Substrat, welches zuvor in 100 µl Puffer gelöst und mit NaOH auf pH 6-7 eingestellt wurde. Die Ansätze wurden im Schüttelschrank bei 42°C und 150 rpm inkubiert. Parallel zu jedem Ansatz wurde ein Blindwert gemessen, wobei identische Reaktionsbedingungen ohne Zusatz von Zellen eingehalten wurden. Da die Kontrollversuche in allen Fällen negativ ausfielen, kann eine spontane Racemisierung bei gegebenen Reaktionsbedingungen ausgeschlossen werden.

Nach ein bzw. zwei Tagen im Schüttelschrank wurden die Eppendorffiolen 5 Minuten lang bei 13000 rmp zentrifugiert und anschließend je 400 µl der überstehenden Lösung entnommen. Die Lösung wurde zweimal mit je 600 µl EE ausgeschüttelt, die organischen Phasen vereinigt, über $Na_2SO_4$ getrocknet und das Lösungsmittel abrotiert. Um eine Trennung der beiden Enantiomere über eine chirale Säule zu erreichen, mussten alle untersuchten Verbindungen derivatisiert werden. Zu diesem Zweck wurde jede Probe mit 500 µl $BF_3$-Methanol versetzt, 10 min bei 60°C geschüttelt und mit 1 ml $CH_2Cl_2$ ausgeschüttelt. Die organische Phase wurde über $Na_2SO_4$ getrocknet und auf eine Volumen von ca. 100 µl eingeengt.

Eine Razemsierungs-Reaktion galt als negativ (-), wenn der Enantiomerenüberschuss nach 2 Tagen > 95% war, Enantiomerenüberschüsse < 20% wurden als positiv (+) bewertet. Alle Übrigen erhielten die Bewertung (~). Die Ergebnisse des Screenings sind in Tabelle 6 zusammengefasst.

Tabelle 6: Ergebnisse des Aktivitätsscreenings

| **Mikroorganismus** | **DSM Nummer** | **Substrat** | | |
|---|---|---|---|---|
| | | ***(S)*-1** | ***(S)*-2** | ***(S)*-3** |
| *L. paracasei* | 20008 | + | + | + |
| *L. paracasei* | 20207 | + | + | + |
| *L. paracasei* | 2649 | - | + | + |
| *L. acidophilus* | 20079 | n.b. | - | - |
| *L. brevis* | 20054 | + | - | - |
| *L. piscicola (L. carnis)* | 20722 | n.b. | - | - |
| *L. halotolerans* | 20190 | + | - | - |
| *L. confusus* | 20196 | - | - | - |
| *L. acetotolerans* | 20749 | + | - | - |
| *L. delbrueckii* | 20074 | + | + | + |
| *L. kandleri* | 20593 | n.b. | - | - |
| *L. fructosus* | 20349 | - | - | - |
| *L. farciminis* | 20184 | - | ~ | - |
| *L. gasseri* | 20243 | n.b. | - | - |
| *L. alimentarius* | 20249 | + | - | - |
| *L. jensenii* | 20557 | + | - | - |
| *L. halofax volcanii* | 5176 | n.b. | - | - |
| *L. haloarcula vallis-mortis* | 3756 | n.b. | - | - |
| + = gute Aktivität - = keine Aktivität ~ = mäßige Aktivität n.b. = nicht bestimmt | | | | |

**Beispiel 3: Bestimmung der Enantlomerenüberschüsse mit verschiedenen Substraten**

Bei der Bestimmung der Enantiomerenüberschüsse wurde wie in Beispiel 2 verfahren. Lediglich die Menge der eingesetzten Zellen wurde auf 50 mg reduziert.

a) Versuche mit L-(-)-3-Phenylmilchsäure als Substrat

Zunächst wurden 50 mg lyophiliserte Zellen mit 1 ml BisTris-Puffer (50 mM, pH6) versetzt und 1 h am Schüttler bei 42˚C und 150 rpm rehydratisiert. Anschließend wurden jeweils 5 mg Substrat gelöst in 100 µl Puffer zugesetzt und Im Schüttelschrank (30˚C, 150 rpm) inkubiert. Alle Proben wurden jeweils nach 24 bzw. 48 Stunden mittels chiralem GC vermessen. Um eine Trennung der Enantiomere am GC zu erzielen, mussten die Proben derivatisiert werde.

Die Derivatisierung erfolgte mit $BF_3$-Methanol (vgl. oben) oder mit Trifluoressigsäureanhydrid. Für die Derivatisierung mit Trifluoressigsäureanhydrid wurden 5mg 3-Phenylmilchsäure In 500µl $CH_2Cl_2$ gelöst, mit 5 Tropfen Trifiuoressigsäureanhydrid versetzt, 1min bei Raumtemperatur geschüttelt, anschließend mit ca. 500µl $H_2O$ versetzt. Die organische Phase wird mit $Na_2SO_4$ getrocknet, danach erfolgt die Vermessung am GC auf chiraler Säule.

Die Berechnung der Ergebnisse erfolgte nach folgenden Formeln:

$$\text{e.e. [\%]: (R-S)/(R+S)} \times 100$$

$$\text{Umsatz [\%]:R/(R+S)} \times 100$$

$$\text{Racemisierung [\%]: 2R/(R+S)} \times 100$$

Neben dem Stamm *Lactobacillus paracasei* subsp. *paracasei* DSM 20008 konnten die folgenden vier weiteren Stämme mit guter bzw. ausgezeichneter Racemaseaktivität bezüglich L-(-)-3-Phenylmilchsäure gefunden werden

- *Lactobacillus paracasei* subsp. *paracasei* DSM 2649
- *Lactobacillus paracasei* subsp. *paracasei* DSM 20207
- *Lactobacillus delbrueckii* subsp. *delbrueckii* DSM 20074
- *Lactobacillus oris* DSM 4864

Die Ergebnisse für obige Stämme sind in folgender Tabelle 7, aufgeschlüsselt nach Kultivierungsbedingungen (Schüttler oder Trockenschrank; Argon oder Luft-Atmosphäre) und Kultivierungsdauer, zusammenfasst.

Tabelle 7: Ergebnisse aus dem Screening mit L(-) Phenylmilchsäure

| Stamm DSM Nummer | Ernte tag | Trockenschrank (Argon) | | | | | | Schüttler (Luft) | | | | | | Trockenschrank (Luft) | | | | | |
|---|---|---|---|---|---|---|---|---|---|---|---|---|---|---|---|---|---|---|---|
| | | 24 h | | | 48 h | | | 24 h | | | 48 h | | | 24 h | | | 48 h | | |
| | | e.e. [%] | Umsatz [%] | Racem. [%] | e.e. [%] | Umsatz [%] | Racem. [%] | e.e. [%] | Umsatz [%] | Racem. [%] | e.e. [%] | Umsatz [%] | Racem. [%] | e.e. [%] | Umsatz [%] | Racem. [%] | e.e. [%] | Umsatz [%] | Racem. [%] |
| 2649 | 3. | 80 | 10 | 20 | 49 | 26 | 51 | 69 | 15 | 31 | 51 | 24 | 49 | 77 | 12 | 23 | 0 | 50 | 100 |
| | 4. | 14 | 43 | 86 | 0 | 50 | 100 | | | | | | | | | | | | |
| 20207 | 3. | 8 | 46 | 92 | 0 | 50 | 100 | | | | | | | | | | | | |
| | 4. | | | | | | | 65 | 17 | 35 | 46 | 26 | 54 | 17 | 41 | 83 | 0 | 50 | 100 |
| 20074 | 3. | 83 | 8 | 16 | 64 | 18 | 36 | - | - | - | - | - | - | 13 | 43 | 87 | 0 | 50 | 100 |
| 4864 | 4. | | | | | | | 23 | 38 | 77 | 0 | 50 | 100 | 70 | 15 | 30 | 45 | 27 | 55 |
| 20008 | 3. | 0 | 50 | 100 | 0 | 50 | 100 | | | | | | | 0 | 50 | 100 | 0 | 50 | 100 |

Um festzustellen, ob die beobachtete Racemase-Aktivität mit einer Lactat-Racemase-Aktivität korreliert, wurde mit den Stämmen, die gute bis sehr gute Aktivität mit L-(-)-3-Phenylmilchsäure zeigten, das natürliche Substrat L-(+)-Milchsäure umgesetzt.

Es zeigte sich, dass bei der Umsetzung des natürlichen Substrates L-(+)-Milchsäure mit den Stämmen DSM 20207, 20074 und 20008 bereits nach 24 Stunden eine 100%ige Racemisierung zu detektieren war.

Im Falle des Stammes DSM 2649 konnte kein Umsatz erzielt werden.

b) Versuche mit verschiedenen weiteren Alkyl-Substraten

Als Substrate wurden verwendet:

(*S*)-(+)-2-Hydroxy-3-methylbutansäure ($C_5H_{10}O_3$)
(*S*)-(-)-2-Hydroxyisocapronsäure ($C_6H_{12}O_3$)

Beide Substrate wurden mit allen Stämmen umgesetzt. Es wurde jeweils nach 24 Stunden und nach 48 Stunden der Racemisierungsgrad mittels GC vermessen.

Im Falle von (*S*)-(+)-2-Hydroxy-3-methylbutansäure zeigt nur der Stamm DSM 20207 Aktivität. Gegenüber dem Substrat (*S*)-(-)-2-Hydroxyisocapronsäure zeigten insgesamt 5 Stämme Racemisierungsaktivität. Die Ergebnisse sind in den Tabellen 8 und 9 zusammengefasst.

Tabelle 8: Screening mit (*S*)-(+)-2-Hydroxy-3-methylbutansäure

| **Stamm** | 24 Stunden | | | 48 Stunden | | |
|---|---|---|---|---|---|---|
| | e.e [%] | Umsatz [%] | Racem. [%] | e.e. [%] | Umsatz [%] | Racem. [%] |
| DS 20207 | 86 | 7 | 14 | 73 | 14 | 27 |

Tabelle 9: Screening mit (*S*)-(-)-2-Hydroxyisocapronsäure

| **Stamm** | 24 Stunden | | | 48 Stunden | | |
|---|---|---|---|---|---|---|
| | e.e [%] | Umsatz [%] | Racem. [%] | e.e. [%] | Umsatz [%] | Racem. [%] |
| DSM 2649 | 88 | 6 | 12 | 75 | 12 | 25 |
| DSM 20008 | 85 | 7 | 15 | 82 | 9 | 18 |
| DSM 20207 | 94 | 3 | 6 | 73 | 14 | 17 |
| DSM 20074 | 94 | 3 | 6 | 93 | 4 | 7 |
| DSM 20017 | 86 | 7 | 14 | 89 | 10 | 20 |

Zusammenfassend kann gesagt werden, dass wieder die gleichen Stämme Aktivität zeigten, die auch gegenüber L-(-)-3-Phenylmilchsäure aktiv waren.

c) Versuche mit (*S*)-2-Hydroxy-4-phenylbuttersäure

Es wurde jeweils nach 24 Stunden und nach 48 Stunden der Racemisierungsgrad mittels HPLC vermessen. Die Ergebnisse sind in Tabelle 10 zusammengefasst.

Tabelle 10: Screening mit (*S*)-2-Hydroxy-4-phenylbuttersäure

| **Stamm** | 24 Stunden | | |
|---|---|---|---|
| | e.e [%] | Umsatz [%] | Racem. [%] |
| DSM 2649 | 6 | 47 | 94 |

(fortgesetzt)

| Stamm | 24 Stunden | | |
|---|---|---|---|
| | e.e [%] | Umsatz [%] | Racem. [%] |
| DSM 20054 | 90 | 5 | 10 |
| DSM 20190 | 70 | 15 | 30 |
| DSM 20008 | 25 | 38 | 75 |
| DSM 20207 | 3 | 48 | 97 |
| DSM 20749 | 48 | 26 | 52 |

Im Zusammenhang mit vorliegender Erfindung wurden die folgenden öffentlich zugänglichen Mikroorganismenstämme der DSMZ nach den Bestimmungen des Budapester Vertrags am 11. Juli 2003 erneut hinterlegt.

| DSM-Nummer | Hinterlegungsnummer |
|---|---|
| 20207 | DSM 15755 |
| 20074 | DSM 15754 |
| 20017 | DSM 15753 |
| 4864 | DSM 15752 |
| 2649 | DSM 15751 |

**Patentansprüche**

1. Verfahren zur mikrobiologischen Isomerisierung von alpha-Hydroxycarbonsäuren der Formel I

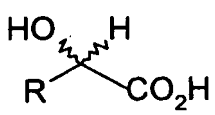

(I)

worin
R für geradkettiges oder verzweigtes $C_2$-$C_8$-Alkyl oder $C_2$-$C_8$-Alkenyl oder - $(CH_2)_n$-Cyc steht, worin n für einen ganzzahligen Wert von 0 bis 4 steht und Cyc für einen gegebenenfalls ein- oder mehrfach substituierten, ein- oder zweikernigen carbo- oder heterocyclischen Ring steht,
wobei man ein Substrat, enthaltend im wesentlichen eine erste stereoisomere Form einer alpha-Hydroxycarbonsäure der Formel (I), mit Hilfe eines enzymhaltigen Extrakts eines Mikroorganismus oder in Gegenwart ganzer Zellen eines Mikroorganismus isomerisiert, wobei der Mikroorganismus aus natürlichen oder rekombinanten Mikroorganismen der Gattung *Lactobacillus* oder *Lactococcus* zur Racemisierung ausgewählt ist, die zur Racemisierung wenigstens einer Verbindung, ausgewählt unter der (R)- und/oder (S)-Form von Phenyllactat, 4-Fluorphenyllactat, 2-Hydroxy-4-phenylbuttersäure, 2-Hydroxy-4-methylpentancarbonsäure und 2-Hydroxy-3-methylbuttersäure befähigt sind, und gegebenenfalls das dabei gebildete Isomerengemisch oder ein gebildetes zweites Stereoisomer isoliert, oder ein gebildetes zweites Stereoisomer aus dem Reaktionsgleichgewicht entfernt.

2. Verfahren nach Anspruch 1 wobei der Mikroorganismus ausgewählt ist unter *L. paracasei, L. delbrueckii, L. sakei* und *L. oris.*

3. Verfahren nach Anspruch 2, wobei der Mikroorganismus ausgewählt ist unter den Stämmen *L. paracasei* DSM 20207 (DSM 15755) und DSM 2649 (DSM 15751), *L. delbrueckii DSM20074* (DSM 15754), *L. sakei DSM* 20017 (DSM 15753 und *L. oris* DSM 4864 (DSM 15752).

4. Verfahren nach einem der Ansprüche 1 bis 3, wobei man aus dem gebildeten Isomerengemisch das gewünschte Stereoisomer im Wesentlichen entfernt und den Rückstand erneut einer Isomerisierung unterzieht.

5. Verfahren nach einem der Ansprüche 1 bis 3, wobei man das gebildete Isomerengemisch einer chemischen oder enzymatischen stereoselektiven Folgereaktion unterzieht und das anfallende Reaktionsgemisch erneut einer Isomerisierung unterzieht.

6. Verfahren nach einem der Ansprüche 1 bis 3, wobei die Isomerisierungsreaktion mit einer chemischen oder enzymatischen, enantioselektiven Folgereaktion koppelt, wobei das gewünschte gebildete Stereoisomer der alpha-Hydroxycarbonsäure aus dem Reaktionsgleichgewicht entfernt wird.

7. Verfahren nach einem der Ansprüche 5 und 6, wobei die chemische oder enzymatische, enantioselektive Folgereaktion ausgewählt ist unter einer Veresterung und einer Amidierung der alpha-Hydroxycarbonsäure.

## Claims

1. A method for the microbiological isomerization of alpha-hydroxycarboxylic acids of the formula I

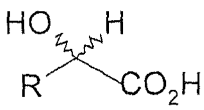

1.

(I)

where
R is straight-chain or branched $C_2$-$C_8$-alkyl or $C_2$-$C_8$-alkenyl or $-(CH_2)_n$-Cyc, where n is an integer from 0 to 4, and Cyc is an optionally mono- or polysubstituted, mono- or binuclear carbo- or heterocyclic ring,
where a substrate comprising essentially a first stereoisomeric form of an alpha-hydroxycarboxylic acid of the formula (I) is isomerized with the aid of an enzyme-comprising extract of a microorganism or in the presence of intact cells of a microorganism, the microorganism being selected from among natural or recombinant microorganisms of the genus *Lactobacillus* or *Lactococcus* for racemization, which microorganisms are capable of racemizing at least one compound selected from among the (R) and/or (S) forms of phenyl lactate, 4-fluorophenyl lactate, 2-hydroxy-4-phenylbutyric acid, 2-hydroxy-4-methylpentanecarboxylic acid and 2-hydroxy-3-methylbutyric acid, and, if appropriate, the resulting isomer mixture or a resulting second stereoisomer is isolated, or a resulting second stereoisomer is removed from the reaction equilibrium.

2. The method according to claim 1, wherein the microorganism is selected from among *L. paracasei, L. delbrueckii*, *L. sakei* and *L. oris.*

3. The method according to claim 2, wherein the microorganism is selected from among the strains *L. paracasei* DSM 20207 (DSM 15755) and DSM 2649 (DSM 15751), *L. delbrueckii* DSM20074 (DSM 15754), *L. sakei* DSM 20017 (DSM 15753) and *L. oris* DSM 4864 (DSM 15752).

4. The method according to any of claims 1 to 3, wherein the desired stereoisomer is essentially removed from the isomer mixture formed and the remainder is subjected to a further isomerization step.

5. The method according to any of claims 1 to 3, wherein the isomer mixture formed is subjected to a chemical or enzymatic stereoselective subsequent reaction and the reaction mixture obtained is subjected to a further isomerization step.

6. The method according to any of claims 1 to 3, wherein the isomerization reaction is coupled with a chemical or enzymatic, enantioselective subsequent reaction, during which reaction the resulting desired stereoisomer of the alpha-hydroxycarboxylic acid is removed from the reaction equilibrium.

7. The method according to claim 5 or 6, wherein the chemical or enzymatic, enantioselective subsequent reaction is selected from among an esterification and an amidation of the alpha-hydroxycarboxylic acid.

## Revendications

1. Procédé pour l'isomérisation biologique d'acides alpha-hydroxycarboxyliques de formule I

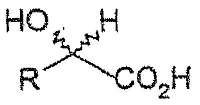

(I)

dans laquelle
R représente un groupe alkyle en $C_2$-$C_8$ ou alcényle en $C_2$-$C_8$ à chaîne droite ou ramifiée ou $-(CH_2)_n$-Cyc, où n représente un nombre entier valant de 0 à 4 et Cyc représente un cycle carbocyclique ou hétérocyclique mono- ou binucléaire, éventuellement une ou plusieurs fois substitué,
dans lequel on isomérise un substrat, contenant essentiellement une première forme stéréoisomère d'un acide alpha-hydroxycarboxylique de formule (I), à l'aide d'un extrait, contenant une enzyme, d'un micro-organisme ou en présence de cellules entières d'un micro-organisme, le micro-organisme étant choisi pour la racémisation parmi des micro-organismes naturels ou recombinants appartenant au genre *Lactobacillus* ou *Lactococcus,* qui sont aptes à la racémisation d'au moins un composé choisi parmi la forme (R) et/ou la forme (S) du lactate de phényle, du lactate de 4-fluorophényle, de l'acide 2-hydroxy-4-phénylbutyrique, de l'acide 2-hydroxy-4-méthylpentacarboxylique et de l'acide 2-hydroxy-3-méthylbutyrique, et éventuellement on isole le mélange d'isomères ainsi formé ou un deuxième stéréoisomère formé, ou on élimine de l'équilibre de la réaction un deuxième stéréoisomère formé.

2. Procédé selon la revendication 1, dans lequel le micro-organisme est choisi parmi *L. paracasei, L. delbrueckii*, *L. sakei et L. oris.*

3. Procédé selon la revendication 2, dans lequel le micro-organisme est choisi parmi les souches L. *paracasei* DSM 20207 (DSM 15755) et DSM 2649 (DSM 15751), *L. delbrueckii* DSM 20074 (DSM 15754), *L. sakei* DSM 20017 (DSM 15753) et *L. oris* DSM 4864 (DSM 15752).

4. Procédé selon l'une quelconque des revendications 1 à 3, dans lequel on sépare en majeure partie le stéréoisomère recherché du mélange d'isomères formé et on soumet à nouveau le résidu à une isomérisation.

5. Procédé selon l'une quelconque des revendications 1 à 3, dans lequel on soumet le mélange d'isomères formé à une réaction chimique ou enzymatique stéréosélective suivante et on soumet à nouveau à une isomérisation le mélange réactionnel résultant.

6. Procédé selon l'une quelconque des revendications 1 à 3, dans lequel la réaction d'isomérisation est couplée à une réaction chimique ou enzymatique énantiosélective suivante, le stéréoisomère recherché, formé, de l'acide alpha-hydroxycarboxylique, étant séparé du mélange réactionnel.

7. Procédé selon la revendication 5 ou 6, dans lequel la réaction chimique ou enzymatique énantiosélective suivante est choisie parmi une estérification et une amidation de l'acide alpha-hydroxycarboxylique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **G.L. Kenyon ; J.A. Gerlt ; G.A. Petsko ; J.W. Kozarich.** Mandelate Racemase: Structure-Function Studies of a Pseudosymmetric Enzyme. *Accts. Chem. Res.,* 1995, vol. 28, 178-186
- **Powers, J.W. Kozarich ; G.L. Kenyon.** Racemization of Vinylglycolate Catalyzed by Mandelate Racemase. *J. Org. Chem.,* 1995, vol. 60, 3347-3351
- **S.S. Schafer ; A.T. Kallarakal ; J.W. Kozarich ; J.A. Gerlt ; J.R. Clifton ; G.L. Kenyon.** Mechanism of the Reaction Catalyzed by Mandelate Racemase: The Structure and Mechanistic Properties of the D270N Mutant. *Biochemistry,* 1996, vol. 35, 5662-5669
- **B. Schnell et al.** Enzymatic Racemisation and its Application to Synthetic Biotranformations. *Adv. Synth. Catal.,* 2003, vol. 345, 653-666
- **U.T. Strauss ; K. Faber.** Deracemization of (±) mandelic acid using a lipase-mandelate racemase two-enzyme system. *Tetrahedron: Assymetry,* 1999, vol. 10, 4079-4081
- **S.-Q Liu.** Practical implications of lactat and pyruvate metabolism by lactic acid bacteria in food and beverage fermentations. *Int. J. Food Microbiol.,* 2003, vol. 83, 115-131
- **S.M. Glück et al.** Lactate Racemase as a versatile tool for the racemazation of $\alpha$-hydroxycarbolic acids. *Chemicke Listy,* 2003, vol. 97 (363), 121
- **Felfer, U. et al.** *J. Mol. Catal. B: Enzymatic,* 2001, vol. 15, 213
- **Pearson ; Lipman.** *Proc. Natl. Acad, Sci. (USA),* 1988, vol. 85 (8), 2444-2448
- **Narang, S.A.** *Tetrahedron,* 1983, vol. 39, 3
- **Itakura et al.** *Annu. Rev. Biochem.,* 1984, vol. 53, 323
- **Itakura et al.** *Science,* 1984, vol. 198, 1056
- **Ike et al.** *Nucleic Acids Res.,* 1983, vol. 11, 477
- **Arkin ; Yourvan.** *PNAS,* 1992, vol. 89, 7811-7815
- **Delgrave et al.** *Protein Engineering,* 1993, vol. 6 (3), 327-331
- **Voet ; Voet.** Phosphoamiditmethode. Wiley Press, 896-897
- **Sambrook et al.** Molecular Cloning: A laboratory manual. Cold Spring Harbor Laboratory Press, 1989
- **Sambrook, J. ; Fritsch, E.F. ; Maniatis, T.** Molecular Cloning: A Laboratory Manual. Cold Spring Harbor Laboratory Press, 1989
- **Sambrook et al.** Molecular Cloning. Cold Spring Harbor Laboratory, 1989
- Current Protocols in Molecular Biology. John Wiley & Sons, 1985
- Nucleic Acids Hybridization: A Practical Approach. IRL Press at Oxford University Press, 1985
- Essential Molecular Biology: A Practical Approach. IRL Press at Oxford University Press, 1991
- **Ausubel et al.** Current Protocols in Molecular Biology. John Wiley & Sons, 1989
- **Goeddel.** Gene Expression Technology: Methods in Enzymology. Academic Press, 1990, vol. 185
- Buch Cloning Vectors. Elsevier, 1985
- **T. Maniatis ; E.F. Fritsch ; J. Sambrook.** Molecular Cloning: A Laboratory Manual. Cold Spring Harbor Laboratory, 1989
- **T.J. Silhavy ; M.L. Berman ; L.W. Enquist.** Experiments with Gene Fusions. Cold Spring Harbor Laboratory, 1984
- **Ausubel, F.M. et al.** Current Protocols in Molecular Biology. Greene Publishing Assoc. and Wiley Interscience, 1987
- Cloning Vectors. Elsevier, 1985
- Current Protocols in Molecular Biology. Wiley Interscience, 1997
- **Sambrook et al.** Molecular Cloning: A Laboratory Manual. Cold Spring Harbor Laboratory Press, 1989
- **Thomas, K.R. ; Capecchi, M.R.** *Cell,* 1987, vol. 51, 503
- Biotechnology. 1993, vol. 3
- **E. J. Ebbers ; G. J. A. Ariaans ; J. P. M. Houbiers ; A. Bruggink ; B. Zwanenburg.** *Tetrahedron,* 1997, vol. 53, 9417-9476
- **U. T. Strauss ; K. Faber.** *Tetrahedron: Asymmetry,* 1999, vol. 10, 4079-4081
- **F. van Rantwijk ; M. A. P. J. Hacking ; R. A. Sheldon.** *Monatsh. Chem. /Chem. Monthly,* 2000, vol. 131, 549-569